# EUROPEAN PATENT APPLICATION

(11) **EP 2 369 008 A1**
(43) Date of publication of application: **28.09.2011**
(21) Application number: 10156717.0
(22) Date of filing: 17.03.2010
(51) Int. Cl.: C12Q 1/68

(54) **IGFBP-3 concentration-related genes**

(71) Applicant: SIEMENS AKTIENGESELLSCHAFT, 80333 München (DE)
(72) Inventor: Friedrich, Nele, Dr., 17489, Greifswald (DE); Biffar, Reiner, Prof., 17498, Neuenkirchen (DE); Ernst, Florian, Dr., 17489, Greifswald (DE); Homuth, Georg, Dr., 17489, Greifswald (DE); Nauck, Matthias, Prof., 17498, Neuenkirchen (DE); Teumer, Alexander, 17489, Greifswald (DE); Völker, Uwe, Prof., 17489, Neuenkirchen (DE); Völzke, Henry, Dr., 17509, Brünzow (DE); Wallaschofski, Henry, Dr., 17489, Greifswald (DE)

(57) **Abstract**

The invention relates to the application of SNPs (single nucleotide polymorphisms) which are associated with altered levels of IGFBP-3 concentration and comprises diagnostic and screening methods using the novel SNP associations described herein.

## Description

The invention relates to the application of SNPs (single nucleotide polymorphisms) which are associated with altered levels of IGFBP-3 concentration and comprises diagnostics and screening methods using the novel SNP associations described herein.

The insulin-like growth factor (IGF) system consists of two ligands (IGF-I and IGF-II), three IGF receptors (IGF-I receptor; IGF-II receptor; insulin receptor), and six IGF-binding proteins (IGFBP-1 to IGFBP-6) which are involved in cell replication and proliferation, protein synthesis, carbohydrate homeostasis, and bone metabolism. Compared to IGF-I, IGFBPs are existing in excess in serum, thus IGF-I is immediately bound. Only a small fraction of IGF-I is unbound or free, which is postulated to be the bioactive form [1]. Circulating IGF-I is mainly bound to IGFBP-3 [2]. Therefore, IGFBP-3 plays a major role in the IGF-system and directly influences the IGF-I concentration. Both increased and decreased IGFBP-3 levels are related to a broad range of diseases:
Decreased IGFBP-3 levels:
   coronary artery disease
   lung disease
   hypothyroidism
   increased mortality
   cancer
Increased IGFBP-3 levels:
   hyperthyroidism

IGFBP-3 concentrations substantially fluctuate between individuals. Factors like growth hormone concentration, age, and gender explain parts of these variations. Furthermore, twin studies reported that the proportion of variance attributable to genetic effects was 60% for the IGFBP-3 concentration [3].

Table 1 shows a selection of SNPs which are related to the IGFBP-3 concentration.

**Table 1. SNPs related to the IGFBP-3 concentration**

| SNP/ polymorphism | nearest gen | | Ref. |
|---|---|---|---|
| rs2132571 | IGFBP3 | decreased circulation IGFB-3 levels in women | [4, 5] |
| rs2132572 | IGFBP3 | decreased circulation IGFB-3 levels in both gender | [4, 5] |
| rs2854744 | IGFBP3 | increased circulation IGFB-3 levels in both gender | [4, 5] |
| rs375577 | GHRL | promoter region of the GHRL gen: increased IGFBP-3 levels | [6] |

The invention relates to:
1. A method of screening for disease, diagnosis, prognosis or prediction of disease associated with altered IGFBP3 levels by identifying SNPs comprising the steps of:
   identifying in a sample of a patient at least one SNP according to the foregoing description, in particular at least one SNP of Table 2.
2. Method of count 1, wherein said disease is at least one of the diseases comprised in the group of coronary artery disease, lung disease, hypothyroidism, cancer, and hyperthyroidism.

Preferably a plurality of SNPs are analyzed in a sample. Preferred combinations are any 2 of SNPS No. 1-6, 9, 10 and 12; all of SNPs No 1-2; all of SNPs No 1-3; all of SNPs No 1-4; all of SNPs No 1-5; all of SNPs No 1-6; all of SNPs No 1-6 and 9, 10 and 12; any of SNPS No 1, 2, 3, 4, 5, or 6 combined with SNP No. 7; any of SNPS No 1, 2, 3, 4, 5, or 6 combined with SNP No. 8; any of SNPS No 1, 2, 3, 4, 5, or 6 combined with SNP No. 10; any of SNPS No 1, 2, 3, 4, 5, or 6 combined with SNP No. 11; SNPs No 7 and 8, SNPs No 7 and 14; any of SNPs 1-6, 9, 10 or 12 combined with SNP No 11; any of SNPs 1-6, 9, 10 or 12 combined with SNP No 13; any of SNPs 1-6, 9, 10 or 12 combined with SNP No 14; any of SNPs 1-6, 9, 10 or 12 combined with SNP No 15; and of SNPs 1-6, 9, 10 or 12 combined with SNP No 16.

Further preferred combinations include the combination of SNPs No 13, 17, and 18; and the combination of SNPs No 16 and 19.

In a preferred embodiment the invention relates to a screening method comprising the steps of:
identifying in a sample of a patient at least one SNP according to the foregoing description, in particular at least one SNP of Table 2 or one of the above combinations.

When said at least one SNP is identified in said sample, said sample is flagged and further diagnostic tests can be performed to diagnose in said patient at least one of the diseases comprised in the group of coronary artery disease, lung disease, hypothyroidism, cancer, and hyperthyroidism.

The invention thus advantageously allows the screening of large numbers of sample wherein only flagged samples indicate the need to perform further, more expensive tests.

The invention further relates to:
3. A kit for identifying a SNP according to count 1, said kit comprising at least one oligonucleotide probe capable of binding to a sequence containing the mutant variant of said SNP.
4. A kit for identifying a SNP according to count 3, said kit comprising at least one oligonucleotide probe capable of binding to a sequence containing the wild type variant of said SNP.
5. A pharmaceutical composition comprising a substance capable of selectively binding at least one of said SNPs or a surrounding sequence region of said SNP, or a substance capable of interacting with a gene product of a gene harboring said SNP, or a substance capable of interacting with a gene harboring said SNP or a gene in a surrounding sequence of said SNP, e.g. by binding, inhibition or activation of said gene, or said gene product, wherein said gene product comprises an mRNA or polypeptide encoded by said gene harboring said SNP or said gene in a sequence surrounding said SNP.
6. Use of said pharmaceutical composition according to count 5, for treating a disease associated with altered IGFBP-3 concentrations, in particular wherein said disease is at least one of the diseases comprised in the group of coronary artery disease, lung disease, hypothyroidism, cancer, and hyperthyroidism.

The term "SNP" relates to point mutations (Single Nucleotide Polymorphism). The term surrounding sequence regions relates to sequence regions within 50 kB, 10kB, or 1kB of the SNP.

The identification may be achieved by methods known in the art, e.g. sequencing, hybridisation, PCR-based methods, RFLP Analysis, micro array based methods (biochips), bead-based methods (e.g. Illumina), mass spectrometry and others.

The identification can be performed from any sample containing genetic material, e.g. blood sample, swab sample, tissue sample, smear sample, biopsy and others.

Up to now, no data regarding the IGFBP-3 concentration are available from genome wide association (GWA) studies. The SNPs presented herein have been identified in a GWA and present new and useful associations of individual SNPs with altered IGFBP-3 concentration. The SNPs shown herein have extremely high significance with regard to association with altered IGFBP-3 concentration. They are therefore useful for diagnostic and screening purposes of diseases associated with altered IGFBP-3 levels, in particular coronary artery disease, lung disease, hypothyroidism, cancer, and hyperthyroidism.

The GWA results presented are based on data from the Study of Health in Pomerania (SHIP,nN = 4310). For the analyses all subjects with missing data for IGFBP-3, diabetes mellitus, and renal diseases were excluded. Thus, the final study population for the present analyses consisted of 3339 subjects.

The analyses revealed several SNPs which are related to IGFBP-3 concentration. The significant SNPs of the invention are displayed in table 2.

**Table 2. Significant SNPs.**

| **SNP** no. | **ProbeSet** | **DBSnpRSId** | **locus** | **P** | **nearest gene** |
|---|---|---|---|---|---|
| 1 | rs700752 | SNP_A-2302476 | p12.3 | 1,950E-08 | TNS3 |
| 2 | rs700753 | SNP_A-2250382 | p12.3 | 1,963E-08 | TNS3 |
| 3 | rs856563 | SNP_A-1989803 | p12.3 | 2,464E-08 | TNS3 |
| 4 | rs856540 | SNP_A-4203478 | p12.3 | 1,104E-07 | TNS3 |
| 5 | rs4724487 | SNP_A-1931444 | p12.3 | 3,074E-07 | TNS3 |
| 6 | rs1917609 | SNP_A-2203817 | p12.3 | 5,961E-07 | TNS3 |
| 7 | rs1742458 | SNP_A-8686398 | p13.3 | 6,501E-07 | HAGH |
| 8 | rs12805390 | SNP_A-8451524 | q22.3 | 9,001E-07 | PDGFD |
| 9 | rs2116643 | SNP_A-8318574 | p12.3 | 9,900E-07 | TNS3 |
| 10 | rs7782135 | SNP_A-8593768 | p12.3 | 1,056E-06 | TNS3 |
| 11 | rs9931988 | SNP_A-8392034 | p13.3 | 1,117E-06 | C16orf73 |
| 12 | rs1549871 | SNP_A-8607120 | p12.3 | 2,605E-06 | TNS3 |
| 13 | rs7809789 | SNP_A-2112350 | p15.3 | 3,188E-06 | STK31 |
| 14 | rs12514127 | SNP_A-4283751 | q23.1 | 3,752E-06 | PRR16 |
| 15 | rs12653659 | SNP_A-4211221 | q23.1 | 4,663E-06 | PRR16 |
| 16 | rs9841682 | SNP_A-8570510 | p22.1 | 4,688E-06 | ZNF619 |
| 17 | rs2216974 | SNP_A-1989196 | p15.3 | 4,752E-06 | STK31 |
| 18 | rs10233834 | SNP_A-4249571 | p15.3 | 7,592E-06 | STK31 |
| 19 | rs6589648 | SNP_A-8596143 | q22.1 | 8,357E-06 | CNTN5 |
| 20 | rs10504109 | SNP_A-1993224 | q11.22 | 9,458E-06 | SNTG1 |

In table 2 all significant SNP's < 10⁻⁵ are presented, by ascending p-values and information about ProbeSet Nr.( Affymetrix Human SNP Array 6.0), gene bank accession no. (DBSnpRSId), chromosome and position on the gene (locus), p-value, and the nearest gene name is included. The probe set nr. and DBSnpRSId represent redundant information allowing clear identification of the respective SNP, for example in the SNP database run by the US National Center for Bioinformatic Information (http://www.ncbi.nlm.nih.gov/projects/SNP/).

Serum IGF-1 and IGFBP-3 concentrations were determined by automated two-site chemiluminescence immunoassays (Nichols Advantage; Nichols Institute Diagnostica GmbH, Bad Vilbel, Germany). In both assays, in the first step biotin labelled monoclonal antibodies were employed as capture, and in the second step the acridinium-ester labelled monoclonal antibodies were used for detection. To determine IGF-1 concentration the samples were acidified to separate IGF-1 from IGF-binding proteins. The analytical sensitivity was 6 ng/mL. The IGF-1 assay has been calibrated against the World Health Organization International Reference Reagent 1988, Insulin-like Growth Factor-1 87/518. The analytical sensitivity of the IGFBP-3 assay was 20 ng/mL. The assay reference standard was analytically prepared with glycosylated recombinant human IGFBP-3. Only one lot of reagents was used for all IGFBP-3 measurements.

Statistical significance was analyzed using continuous IGFBP-3 levels (i.e. strong deviation correlating to increased significance/lower p-Value). Correlation of increased or decreased IGFBP-3 levels, respectively, and the genotypes was tested for statistical significance in a linear fashion with frequency of risk alleles/SNPs.

Within the region of the IGFBP3 gene several highly significant SNPs were found. Our initial analyses confirmed the already identified SNP rs2132571 which was highly correlated to the already known non-synonymous SNP rs2854746 in an exon of the gene IGFBP3, therefore, we adjusted our analyses for rs2854746 to identify SNPs which are independent of rs2854746. The results of this analysis are shown in Table 2.

Significant SNP were identified in or in the region of the following genes:
TNS3 (tensin 3):
   May play a role in actin remodeling. Involved in the dissociation of the integrin- tensin-actin complex. EGF activates TNS4 and down-regulates TNS3 which results in capping the tail of ITGB1. Seems to be involved in mammary cell migration. May be involved in cell migration and bone development (By similarity)
HAGH (hydroxyacylglutathione hydrolase):
   The enzyme encoded by this gene is classified as a thiolesterase and is responsible for the hydrolysis of S-lactoyl-glutathione to reduced glutathione and D-lactate.
PDGFD (platelet derived growth factor D):
   The protein encoded by this gene is a member of the platelet-derived growth factor family. The four members of this family are mitogenic factors for cells of mesenchymal origin and are characterized by a core motif of eight cysteines, seven of which are found in this factor. This gene product only forms homodimers and, therefore, does not dimerize with the other three family members. It differs from alpha and beta members of this family in having an unusual N-terminal domain, the CUB domain. Two splice variants have been identified for this gene.
STK31 (serine/threonine kinase 31):
   This gene is similar to a mouse gene that encodes a putative protein kinase with a tudor domain, and shows testis-specific expression. Alternative splicing results in multiple transcript variants encoding different isoforms.
PRR16 (proline rich 16): no information
ZNF619 (zinc finger protein 619):
   no information - may be involved in transcriptional regulation
CNTN5 (contactin 5):
   The protein encoded by this gene is a member of the immunoglobulin superfamily. It is a glycosylphosphatidylinositol (GPI)-anchored neuronal membrane protein that functions as a cell adhesion molecule. It may play a role in the formation of axon connections in the developing nervous system. Two alternatively spliced transcript variants encoding different isoforms have been described for this gene.
SNTG1 (syntrophin, gamma 1):
   The protein encoded by this gene is a member of the syntrophin family. Syntrophins are cytoplasmic peripheral membrane proteins that typically contain 2 pleckstrin homology (PH) domains, a PDZ domain that bisects the first PH domain, and a C-terminal domain that mediates dystrophin binding. This gene is specifically expressed in the brain. Transcript variants for this gene have been described, but their full-length nature has not been determined.

None of the mentioned genes were known to be related to a pathophysiological mechanism in IGFBP-3 regulation.

The SNPs disclosed in Table 2 are highly significant for individuals with altered IGFBP-3 concentration. The SNPs disclosed in Table 2 may be used to screen individuals for need of further diagnostic procedures, e.g. for diagnosis of coronary artery disease, lung disease, hypothyroidism, cancer, hyperthyroidism. Determining the presence of one or a plurality of SNPs of Table 2 in a patient sample is indicative of the patient being at risk of having or developing the above mentioned conditions or diseases.

References:
1. Juul A. Serum levels of insulin-like growth factor I and its binding proteins in health and disease. Growth Horm IGF Res 2003; 13: 113-170.
2. Jones JI, Clemmons DR. Insulin-like growth factors and their binding proteins: biological actions. Endocr Rev 1995; 16: 3-34.
3. Harrela M, Koistinen H, Kaprio J, Lehtovirta M, Tuomilehto J, Eriksson J, Toivanen L, Koskenvuo M, Leinonen P, Koistinen R, Seppala M. Genetic and environmental components of interindividual variation in circulating levels of IGF-I, IGF-II, IGFBP-1, and IGFBP-3. J Clin Invest 1996; 98: 2612-2615.
4. Al-Zahrani A, Sandhu MS, Luben RN, Thompson D, Baynes C, Pooley KA, Luccarini C, Munday H, Perkins B, Smith P, Pharoah PD, Wareham NJ, Easton DF, Ponder BA, Dunning AM. IGF1 and IGFBP3 tagging polymorphisms are associated with circulating levels of IGF1, IGFBP3 and risk of breast cancer. Hum Mol Genet 2006; 15: 1-10.
5. Canzian F, McKay JD, Cleveland RJ, Dossus L, Biessy C, Rinaldi S, Landi S, Boillot C, Monnier S, Chajes V, Clavel-Chapelon F, Tehard B, Chang-Claude J, Linseisen J, Lahmann PH, Pischon T, Trichopoulos D, Trichopoulou A, Zilis D, Palli D, Tumino R, Vineis P, Berrino F, Bueno-de-Mesquita HB, van Gils CH, Peeters PH, Pera G, Ardanaz E, Chirlaque MD, Quiros JR, Larranaga N, Martinez-Garcia C, Allen NE, Key TJ, Bingham SA, Khaw KT, Slimani N, Norat T, Riboli E, Kaaks R. Polymorphisms of genes coding for insulin-like growth factor 1 and its major binding proteins, circulating levels of IGF-I and IGFBP-3 and breast cancer risk: results from the EPIC study. Br J Cancer 2006; 94: 299-307.
6. Dossus L, McKay JD, Canzian F, Wilkening S, Rinaldi S, Biessy C, Olsen A, Tjonneland A, Jakobsen MU, Overvad K, Clavel-Chapelon F, Boutron-Ruault MC, Fournier A, Linseisen J, Lukanova A, Boeing H, Fisher E, Trichopoulou A, Georgila C, Trichopoulos D, Palli D, Krogh V, Tumino R, Vineis P, Quiros JR, Sala N, Martinez-Garcia C, Dorronsoro M, Chirlaque MD, Barricarte A, van Duijnhoven FJ, Bueno-de-Mesquita HB, van Gils CH, Peeters PH, Hallmans G, Lenner P, Bingham S, Khaw KT, Key TJ, Travis RC, Ferrari P, Jenab M, Riboli E, Kaaks R. Polymorphisms of genes coding for ghrelin and its receptor in relation to anthropometry, circulating levels of IGF-I and IGFBP-3, and breast cancer risk: a case-control study nested within the European Prospective Investigation into Cancer and Nutrition (EPIC). Carcinogenesis 2008; 29: 1360-1366.

## Claims

1. Method of screening for disease, diagnosis, prognosis or prediction of disease associated with altered IGFBP-3 concentrations by identifying single nucleotide polymorphisms (SNP's) comprising the steps of:
identifying in a sample of a patient at least one SNP according to the foregoing description, in particular at least one SNP of Table 2.

2. Method of claim 1, wherein said disease is at least one of the diseases comprised in the group of coronary artery disease, lung disease, hypothyroidism, cancer, and hyperthyroidism.

3. Kit for identifying a SNP according to claim 1, said kit comprising at least one oligonucleotide probe capable of binding to a sequence containing the mutant variant of said SNP.

4. Kit for identifying a SNP according to claim 2, said kit comprising at least one oligonucleotide probe capable of binding to a sequence containing the wild type variant of said SNP.

5. Pharmaceutical composition comprising a substance capable of selectively binding at least one of said SNPs or a surrounding sequence region of said SNP, or a substance capable of interacting with a gene product of a gene harboring said SNP, or a substance capable of interacting with a gene harboring said SNP or a gene in a surrounding sequence of said SNP, e.g. by binding, inhibition or activation of said gene, or said gene product, wherein said gene product comprises an mRNA or polypeptide encoded by said gene harboring said SNP or said gene in a sequence surrounding said SNP.

6. Use of said pharmaceutical composition according to claim 5, for treating a disease associated with altered IGFBP-3 concentrations, in particular wherein said disease is at least one of the diseases comprised in the group of coronary artery disease, lung disease, hypothyroidism, cancer, and hyperthyroidism.
